Europäisches Patentamt

European Patent Office

Office européen des brevets

(19)

(11) Veröffentlichungsnummer: **0 018 348**
A1

(12) # EUROPÄISCHE PATENTANMELDUNG

(21) Anmeldenummer: 80890025.2

(22) Anmeldetag: 28.02.80

(51) Int. Cl.³: **C 02 F 11/00**, A 23 K 1/00,
C 13 K 1/02, C 12 N 1/00,
A 23 K 1/12

(30) Priorität: 01.03.79 AT 1550/79

(71) Anmelder: **Ruthner, Othmar, Dr. Dipl.-Ing. techn.,**
**Sieveringer Strasse 150, A-1190 Wien (AT)**

(43) Veröffentlichungstag der Anmeldung: 29.10.80
**Patentblatt 80/22**

(72) Erfinder: **Ruthner, Othmar, Dr. Dipl.-Ing. techn.,**
**Sieveringer Strasse 150, A-1190 Wien (AT)**

(84) Benannte Vertragsstaaten: **BE CH DE FR GB IT LU NL**
**SE**

(74) Vertreter: **Köhler-Pavlik, Johann, Dipl.-Ing.,**
**Margaretenplatz 5, A-1050 Wien (AT)**

(54) **Verfahren und Vorrichtung zur Aufbereitung von Abfallstoffen.**

(57) Die Abfallstoffe werden in einem Wirbelschichtreaktor B mit einem gasförmigen Gemisch aus Chlorwasserstoff und Wasserdampf von 110° bis 250° C unter Druck und bei erhöhter Temperatur behandelt. Die Abfallstoffe, welche Müll, Klärschlamm oder zellulosehaltige Stoffe, wie Papier, Pappe und Holz oder Gemische dieser Stoffe sein können, werden dabei sterilisiert und teilweise hydrolysiert. Nach dieser Behandlung wird das Gut in trockener, pulverartiger Form aus dem Wirbelschichtreaktor B abgezogen. An der Gasphase wird durch Regelung des Wasserhaushaltes die gewünschte Zusammensetzung des Behandlungsgases eingestellt und dieses ein- oder mehrmals unter Druck in den Wirbelschichtreaktor B rückgeführt, worauf das erhaltene Trockengut einer weiteren Verwendung bzw. Behandlung einbezogen wird.

- 1 -

Aufbereitung von Müll- und Klärschlamm

Die Erfindung betrifft ein Verfahren zur Aufbereitung von zerkleinertem Müll und/oder Klärschlamm und/oder zellulosehaltige Stoffe wie Papier, Pappe und Holz .

Im Rahmen des Umweltschutzes nimmt die Aufbereitung von Müll-und Klärschlamm eine Schlüsselstellung ein. Es fehlt nicht an Verfahren, wie Kompostierung, Pyrolyse und Verbrennung, um die anfallenden Abfallmassen unter Kontrolle zu bringen. Zumeist geht aber die dem Abfall innewohnende Wärmeenergie für die Nutzung verloren.

Erfindungsgemäß wird ein Verfahren vorgeschlagen, um den organischen Anteil von Müll und Klärschlamm, welcher einen Energiegehalt von ca. 16.000 - 20.000 KJ/kg aufweist, durch Hydrolyse zu erhalten und nach entsprechenden Reinigungsprozessen z.B. für eine Tierfütterung zu verwenden oder einer sonstigen chemischen Verwertung zuzuführen.

Das der Erfindung zugrundeliegende Problem ist natürlich seit geraumer Zeit bekannt und es hat nicht an Versuchen gefehlt, es zu lösen. So wurde gemäß der US-PS 4,119.495 (entspricht der DD-PS 129.152) bereits vorgeschlagen, Klärschlamm mit Säuren bei erhöhter Temperatur zu hydrolysieren und die abgetrennten Flüssiganteile biochemisch weiterzubehandeln.

Weiters zählt es zum bekannten Stand der Techni, Eiweißmaterialien, welche neben den Proteinen noch Stärke und Zellulose enthalten, vor einer mikrobiologischen Behandlung zu hydrolysieren, man vergl. hiezu etwa die GB-PS 1,465.428 sowie die AU-PS 493.531 und 475.824.

Von dem genannten Stand der Technik geht die vorliegende Erfindung aus.

Sie betrifft demgemäß ein Verfahren der eingangs erwähnten Art, dadurch gekennzeichnet, daß das Müll/Klärschlammgemisch in einem Wirbelschichtreaktor mit einem gasförmigen Gemisch aus Chlorwasserstoff und Wasserdampf von 110° bis 250° C unter Druck und bei erhöhter Temperatur behandelt wird, wobei das Gut sterilisiert und teilweise hydrolisiert wird, und daß nach dieser Behandlung das behandelte Gut in trockener, pulverartiger Form aus dem Wirbelschichtreaktor abgezogen wird, während an der vorzugsweise im Kreislauf geführten Gasphase durch Wasserabscheidung bzw. Ergänzung die gewünschte Zusammensetzung des Behandlungsgases eingestellt wird un dieses ein- oder mehrmals unter Druck in den Wirbelschichtreaktor rückgeführt wird, worauf das erhaltene Trockengut einer weiteren Verwendung bzw. Behandlung einbezogen wird.

Bevorzugte Ausgestaltungen dieses Verfahrens bestehen darin, daß das erhaltene Trockengut einer oder mehreren Extraktionen unterzogen wird, wobei einerseits ein Extrakt und andererseits extraktieres Restmaterial erhalten wird; weiters darin, daß der Extrakt zur Entfernung

0018348

von unerwünschten anorganischen Anionen bzw. Kationen, insbesondere von schädlichen Metallionen über Ionenaustauscher geleitet wird und daß dieses gereinigte Produkt zur Erzeugung von SCP (single cell protein) fermentiert wird, bzw. darin, daß die Behandlung der Müll- und/oder Klärschlamm mit azeotroper Salzsäure in einem geschlossenen Kreislauf durchgeführt und unverwertbare Restsubstanzen als Brennstoff zur Deckung des Wärmebedarfs des Hydrolysevorganges verwendet werden.

Weiters betrifft die vorliegende Erfindung auch eine Einrichtung zur Durchführung des genannten Verfahrens. Diese ist im wesentlichen dadurch gekennzeichnet, daß sie einen Wirbelschichtreaktor und eine diesem nachgeschaltete aus Extraktor, gegebenenfalls Ionentauscher und Fermentator bestehende Verarbeitungsanlage umfaßt und daß der Wirbelschichtreaktor Teil eines Kreislaufsystems für die Reaktionsflüssigkeit ist.

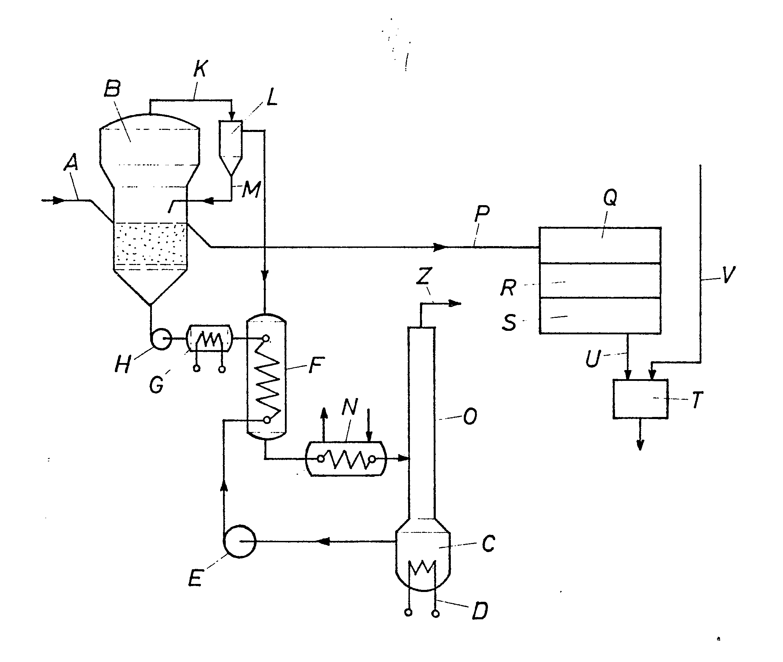

In der Zeichnung ist in Form eines Blockschemas eine Einrichtung zur Durchführung des erfindungsgemäßen Verfahrens gezeigt.

Mit A ist eine Förderanlage bezeichnet, welche dem Wirbelschichtreaktor B vorgeschaltet ist. Der Wirbelschichtreaktor B liegt in einem Kreislaufsystem für die Reaktionsflüssigkeit - z.B. Salzsäure -, welche in dampfförmigem, azeotropen Zustand seinem unteren Teil zugeführt wird. Dieses Kreislaufsystem besteht aus einem Verdampfer C mit einer Heizeinrichtung D, einer Pumpe (bzw. einem Kompressor) E, einem Wärmetauscher F, ggf. einem Erhitzer und einem zusätzlichen Kompressor H, dessen Druckleitung in den unteren Teil des Reaktors mündet.

Vom obersten Abschnitt des Reaktors geht eine Leitung K für abziehendes Dampfgemisch aus. In dieser Leitung K kann ein Zyklon L liegen, um das Dampfgemisch von Staubanteilen zu befreien, die über die Leitung M dem Reaktor B wieder zugeführt werden können. Die Ausgangsseite des Zyklons L ist über den Wärmetauscher F und einen Kühler N mit einer Kolonne O verbunden, welche dem Verdampfer C zugeordnet ist.

Der Innenraum des Reaktors B ist über eine Austrag- und Förderanlage P mit einer Extraktionsanlage Q sowie ggf. mit einem Ionenaustauscher R und einer Fermentationsanlage S verbunden.

Der Fermentationsanlage S, welche an sich die letzte Stufe der erfindungsgemäßen Anlage bildet, kann eine Mischvorrichtung T nachgeschaltet sein, welcher über die Leitung U der Ausstoß der Fermentationsanlage und über eine weitere Leitung V Zusatzmaterial, etwa Grünfutter, zugeführt werden kann.

Im einzelnen besteht das erfindungsgemäße Verfahren darin, daß die Rohsubstanz aus Müll- und Klärschlamm, welche außer Fetten, Proteinen, Rohfaser auch anorganische Bestandteile, Metallverbindungen, etc. enthält, nach einem Siebungs- und Trocknungsprozeß über die Förderanlage A in den Wirbelschichtreaktor B kontinuierlich oder diskontinuierlich eingetragen wird, hiebei mit einem azeotropen, dampfförmigen HCl-Wassergemisch zwischen 120-250°C bei entsprechender Temperatur und dem für die Wirbelschichtreaktion erforderlichen Druck sterilisiert und hydrolisiert wird. Die im Wirbelschichtreaktor anfallende Trockenmasse wird zur weiteren Verarbeitung mittels der Förderanlage P der Anlage Q zugeführt und dort extrahiert. Der Extrakt wird durch Überleiten über den Ionenaustauscher R von Metallen gereinigt und die reinen Extrakte werden in der Anlage S einer Fermentation unterzogen. Das anfallende organische Produkt kann in Form von SCP (Single-cell-proteinen) als KRaftfutter verwertet werden.

0018348

Es ist möglich, dieses KRaftfutter in der Mischvorrichtung T mit Grünfutter zu vermischen und als Tierfutter zu verwenden.

Das aus dem Reaktor B abdampfende azeotrope Dampfgemisch kann gegebenenfalls durch ein angebrachtes Zyklonsystem L von restlichen Staubteilen befreit und die abgeschiedenen Staubmengen können über die Leitung M wieder in den Reaktionsraum des Wirbelschichtreaktors B zurückgeführt werden. Der azeotrope Säuredampf wird über den Wärmetauscher F und den Kühler N in die Kolonne O gebracht, in welcher das flüssige Azeotrop vom aufgenommenen Wasser im Verdampfer C getrennt wird. Mit Hilfe eines Dephlemators wird das Wasser abgeschieden; es wird über die Leitung Z abgeführt. Das azeotrope Gemisch wird nach der Erhitzung im Verdampfer C auf etwa 120°C mittels der Pumpe (bzw. Kompressor) E über den Wärmetauscher F und den zusätzlichen Erhitzer G sowie nach zusätzlicher Kompression im Kompressor H in dampfförmigem Zustand unter Druck und bei einer Temperatur von ca. 120 - 300°C wieder in den Wirbelschichtreaktor B eingepreßt.

Das erfindungsgemäße Verfahren kann natürlich im Rahmen der vorliegenden Erfindung auch auf verwandte Stoffe und Produkte wie z.B. zellulosehaltige Stoffe oder vegetabile Stoffe angewendet werden.

- 6 -

0018348

Patentansprüche

1. Verfahren zur Aufbereitung von zerkleinertem Müll und/oder Klärschlamm und/oder zellulosehaltigen Stoffe, wie Papier, Pappe und Holz, dadurch gekennzeichnet, daß das Müll/Klärschlammgemisch in einem Wirbelschichtreaktor (B) mit einem gasförmigen Gemisch aus Chlorwasserstoff und Wasserdampf von 110° bis 250°C unter Druck und bei erhöhter Temperatur behandelt wird, wobei das Gut sterilisiert und teilweise hydrolisiert wird, und daß nach dieser Behandlung das behandelte Gut in trockener, pulverartiger Form aus dem Wirbelschichtreaktor (B) abgezogen wird, während an der vorzugsweise im Kreislauf geführten Gasphase durch Wasserabscheidung bzw. Ergänzung die gewünschte Zusammensetzung des Behandlungsgases eingestellt wird und dieses ein- oder mehrmals unter Druck in den Wirbelschichtreaktor (B) rückgeführt wird, worauf das erhaltene Trockengut einer weiteren Verwendung bzw. Behandlung einbezogen wird.

2. Verfahren nach Anspruch 1, dadurch gekennzeichnet, daß das erhaltene Trockengut einer oder mehreren Extraktionen unterzogen wird, wobei einerseits ein Extrakt und andererseits extraktiertes Restmaterial erhalten wird.

3. Verfahren nach Anspruch 2, dadurch gekennzeichnet, daß der Extrakt zur Entfernung von unerwünschten anorganischen Anionen bzw. Kationen, insbesondere von schädlichen Metallionen über Ionentauscher geleitet wird und daß dieses gereinigte Produkt zur Erzeugung von SCP (single cell protein) fermentiert wird.

4. Verfahren nach Anpsruch 1, dadurch gekennzeichnet, daß die Behandlung der Müll- und/oder Klärschlämme mit azeotroper Salzsäure in einem geschlossenen Kreislauf durchgeführt und unverwertbare Restsubstanzen als Brennstoff zur Deckung des Wärmebedarfs des Hydrolysevorganges verwendet werden.

0018348

5. Einrichtung zur Durchführung des Verfahrens nach einem oder mehreren der Ansprüche 1 bis 4, dadurch gekennzeichnet, daß sie einem Wirbelschichtreaktor (B) und eine diesem nachgeschaltete, aus Extraktor (Q), ggf. Ionentauscher (R) und Fermentator (S) bestehende Verarbeitungsanlage umfaßt und daß der Wirbelschichtreaktor (B) Teil eines Kreislaufsystems für die Reaktionsflüssigkeit ist.

6. Einrichtung nach Anspruch 5, dadurch gekennzeichnet, daß das Kreislaufsystem einen Verdampfer (C) mit Kolonne (O) und zumindest einen Wärmetauscher (F, G, N) aufweist.

7. Einrichtung nach Anspruch 6, dadurch gekennzeichnet, daß der Wärmetauscher (F) einerseits in einer zum Reaktor (B) führenden Leitung für die Reaktionsflüssigkeit, anderseits in einer Leitung (L) für die vom Reaktor (B) zur Kolonne (O) strömenden Dämpfe liegt.

8. Einrichtung nach Anspruch 6, dadurch gekennzeichnet, daß in der vom Reaktor (B) zum Wärmetauscher (F) führenden Leitung (K) ein Zyklon (L) liegt, dessen Ableitung in den Reaktor (B) mündet.

| | **EINSCHLÄGIGE DOKUMENTE** | | KLASSIFIKATION DER ANMELDUNG (Int.Cl. ) |
|---|---|---|---|
| Kategorie | Kennzeichnung des Dokuments mit Angabe, soweit erforderlich, der maßgeblichen Teile | betrifft Anspruch | |
| X | AT - B - 530 370 (RUTHNER OTHMAR) (25.05.1979) <br><br> * Seite 2, Zeile 52 bis Seite 6, Zeile 25; Ansprüche 1-10; Figuren 1,2 * <br><br> & AT - A - 6689/76 (15.10.1978) <br><br> -- | 1-7 | C 02 F 11/00 <br> A 23 K 1/00 <br> C 13 K 1/02 <br> C 12 N 1/00 <br> A 23 K 1/12 |
| X | DE - A - 2 632 028 (HANS KNAUTH) <br> * Seite 4, Zeile 1 bis Seite 7, Ende; Figur 1; Ansprüche 1-3 * <br><br> -- | 1,2,4-7 | RECHERCHIERTE SACHGEBIETE (Int. Cl. ) |
| A | GB - A - 127 338 (THE MOCASSINE CO. LTD.) <br><br> -- | | C 02 F 11/00 <br> 11/18 <br> 1/02 <br> C 12 N 1/00 <br> 1/22 <br> D 21 C 3/04 <br> C 13 K 1/02 <br> A 23 K 1/12 <br> 1/00 |
| A | CHEMICAL ABSTRACTS, Band 84, Nr. 1, 5. Januar 1976, Zusammenfassung Nr. 3303w, Seite 300, Columbus, Ohio, US, <br> & SU - A - 485 144 (ALL-UNION SCIENTIFIC-INDUSTRIAL ENTER-PRISES OF THE MICROBIOLOGICAL INDUSTRY)(25.09.1975) <br><br> -- | | |
| A | DE - A - 1 567 339 (F.H. FUNK) <br><br> ---- | | |

X | Der vorliegende Recherchenbericht wurde für alle Patentansprüche erstellt.

| Recherchenort | Abschlußdatum der Recherche | Prüfer |
|---|---|---|
| Den Haag | 25-06-1980 | DESCAMPS |

EPA form 1503.1  06.78